(19) Europäisches Patentamt
European Patent Office
Office européen des brevets

(11) EP 1 061 965 B1

(12) # FASCICULE DE BREVET EUROPEEN

(45) Date de publication et mention de la délivrance du brevet:
**14.05.2008 Bulletin 2008/20**

(21) Numéro de dépôt: **99907694.6**

(22) Date de dépôt: **12.03.1999**

(51) Int Cl.:
***A61L 15/58*** *(2006.01)*

(86) Numéro de dépôt international:
**PCT/FR1999/000553**

(87) Numéro de publication internationale:
**WO 1999/045977 (16.09.1999 Gazette 1999/37)**

(54) **MASSE ADHESIVE HYDROCOLLOIDE UTILISABLE A DES FINS MEDICALES**

HYDROCOLLOID ENTHALTENDE KLEBEMASSE FÜR MEDIZINISCHE ANWENDUNGEN

HYDROCOLLOID ADHESIVE MASS USEFUL FOR MEDICAL PURPOSES

(84) Etats contractants désignés:
**AT BE CH CY DE DK ES FI FR GB GR IE IT LI LU MC NL PT SE**

(30) Priorité: **12.03.1998 FR 9803043**

(43) Date de publication de la demande:
**27.12.2000 Bulletin 2000/52**

(73) Titulaire: **Laboratoires Urgo**
**21300 Chenove (FR)**

(72) Inventeurs:
- **AUGUSTE, Stéphane**
**F-21800 Quetigny (FR)**
- **APERT, Laurent**
**F-21000 Dijon (FR)**
- **GARIMA, Luc**
**F-21000 Dijon (FR)**

(74) Mandataire: **Hubert, Philippe**
**Cabinet Beau de Loménie**
**158, rue de l'Université**
**75340 Paris Cédex 07 (FR)**

(56) Documents cités:
**EP-A- 0 355 536**
**EP-A- 0 601 463**
**WO-A-91/06290**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

### Domaine de l'invention

La présente invention a pour objet de nouvelles masses adhésives hydrocolloïdes présentant une absorption accrue lors des premières heures d'utilisation.

L'invention concerne également l'utilisation de ces nouvelles masses adhésives hydrocolloïdes à des fins médicales dermatologiques ou cosmétologiques en particulier pour la réalisation de pansements pour le traitement de l'ampoule, des plaies exsudatives, brûlures et des lésions dermo-épidermiques superficielles, profondes, chroniques ou aiguës.

Le traitement des lésions dermo-épidermiques superficielles, profondes, chroniques ou aiguës, des brûlures et en particulier des plaies exsudatives est un problème complexe auquel les pansements développés au cours de ces dernières années n'ont pas apporté de solutions totalement satisfaisantes.

La perte de substances provoquée par une compression mécanique qui crée une ischémie tissulaire ou causée par les problèmes d'origine vasculaire (irrigation, pression etc...), par une blessure (ablation de tissus, chairs, etc...), un abcès, une brûlure est appelée de façon générale exsudation d'une plaie. Le phénomène d'exsudation correspond à l'excrétion des liquides biologiques produits par la plaie tout au long du processus de cicatrisation.

L'exsudation est d'origine sanguine, elle est contrôlée par les médiateurs de la réaction inflammatoire (vasodilatation et vasoconstriction) avec passage de ces liquides au travers des membranes vasculaires. Ces liquides vont alors inonder le lit de la plaie et constituer les éléments favorables à la dégradation des tissus sains péri-lésionnels avec en particulier des phénomènes de macération et des risques de surinfection.

Le rôle d'un pansement est d'absorber ces liquides, tout en maintenant au contact de la plaie un environnement humide favorable aux processus de cicatrisation.

Le pansement optimal doit donc être capable d'absorber ces exsudats tout au long de la cicatrisation de la plaie.

Suivant la gravité de la plaie, ce processus de cicatrisation peut s'étendre de quelques jours pour les plaies très peu exsudatives à plusieurs mois. Ceci implique de renouveler le pansement fréquemment afin de maintenir son potentiel d'absorption et son efficacité.

Afin de limiter au maximum les renouvellements de pansements qui sont traumatisants pour le malade et pour la qualité de la cicatrisation (arrachage des tissus néoformés, saignements, douleur...) on utilise aujourd'hui des pansements dits « hydrocolloïdes » à base de masses adhésives hydrocolloïdes pour maintenir un potentiel d'absorption sur 2 à 4 jours.

Ces masses adhésives hydrocolloïdes qualifiées aussi d'hydrophiles sont formées principalement d'une matrice adhésive, constituée en général d'au moins un élastomère choisi parmi les polymères tels que les polyisobutylènes ou des copolymères séquencés poly(styrène-oléfine-styrène), associés ou non à des agents permettant d'améliorer l'adhésion tels que des résines poisseuses dites tackifiantes, des plastifiants tels que des polybutènes ou des huiles plastifiantes, ou à des agents permettant d'améliorer la cohésion tels que des caoutchoucs butyle etc..., et d'un ou plusieurs hydrocolloïdes.

De nombreuses masses adhésives hydrocolloïdes employées dans la réalisation de tels pansements ont déjà été décrites. On peut ainsi citer par exemple le brevet US 3 972 328, les demandes de brevet suivantes : FR-A-2 495 473, EP-A-130 061 et EP-A-302 536.

Mais aucun de ces documents n'aborde un autre point important de l'exsudation, son aspect cinétique au cours du temps.

On sait en effet que le phénomène exsudatoire est toujours plus important au début de la cicatrisation car c'est là qu'intervient la phase critique de la réaction inflammatoire. Cette réaction inflammatoire est la plus importante durant les 4 premières heures. Il est alors urgent de rétablir l'équilibre hémostatique de la plaie, et donc d'augmenter la capacité d'absorption durant les premières heures. En effet, plus rapidement cet équilibre sera atteint moins la plaie libérera d'exsudats et plus le potentiel d'absorption du pansement sur plusieurs jours sera maintenu à un niveau élevé. On pourra ainsi espacer les renouvellements de pansements et éviter les inconvénients déjà cités qui y sont liés.

De même à chaque renouvellement du pansement lors des soins, nettoyage et désinfection de la plaie et avant la pose d'un nouveau pansement, cette dernière se retrouve face à un environnement plus agressif (contact de l'air, perte d'humidité etc...). Ceci entraîne à nouveau une reprise de la réaction inflammatoire qui va induire une augmentation des exsudats produits. D'où la nécessité dans ce cas aussi d'augmenter la capacité d'absorption durant les premières heures.

Un pansement idéal devrait donc être adapté à la quantité mais aussi au débit d'exsudats produits.

Pour adapter la capacité d'absorption au débit on pourrait penser augmenter la quantité d'hydrocolloïde qui confère à ces masses adhésives leur propriété d'absorption. Mais si on incorpore trop de produits de ce type dans la masse adhésive ces derniers vont gonfler proportionnellement à leur concentration et altérer les propriétés physiques du pansement ce qui entraîne une perte de cohésion et conduit au démantèlement du pansement lors du retrait voire

à sa perte. Cette solution ne saurait par conséquent être satisfaisante puisqu'on retrouve dans ce cas les problèmes liés aux renouvellements et à la durée de vie du pansement.

**Buts de l'invention**

Dans ces conditions, la présente invention a pour but de résoudre le nouveau problème technique consistant en là fourniture d'une masse adhésive hydrocolloïde d'une nouvelle composition qui présente une absorption élevée dès les premières heures sans diminution de ses propriétés de cohésion, d'adhésion et de maniabilité à moyen et long terme.

**Objets de l'invention**

Il a été découvert, et ceci constitue le fondement de la présente invention, qu'il était possible de résoudre ce problème technique d'une façon entièrement satisfaisante et simple de mise en oeuvre, en incorporant à une masse adhésive hydrocolloïde traditionnelle un ester d'acide gras de sorbitan éthoxylé, et de préférence un monoester.

Par ailleurs, il a été constaté que l'addition d'un ester d'acide gras de sorbitan éthoxylé a une masse adhésive hydrocolloïde traditionnelle en particulier une masse adhésive comprenant un élastomère de type poly(styrène-isoprène-styrène), permet d'augmenter de façon significative la capacité de ladite masse à évacuer les liquides absorbés, par augmentation de la perméabilité à la vapeur d'eau. De ce fait, les nouvelles masses hydrocolloïdes conformes à l'invention permettent de réaliser des pansements susceptibles d'éliminer les liquides absorbés et, par conséquent, de maintenir un équilibre absorption-éliminination et un environnement humide favorable à la cicatrisation.

Ainsi, selon un premier aspect, la présente invention a généralement pour objet une masse adhésive hydrocolloïde, constituée d'un hydrocolloïde et d'une matrice adhésive auxquels sont associés ou non un polymère acrylate ayant une température de transition vitreuse inférieure à - 20 °C, et d'un ester d'acide gras éthoxylé.

Plus précisément la présente invention a pour objet une masse adhésive hydrocolloïde notamment utilisable à des fins médicales, caractérisée en ce que ladite masse adhésive hydrocolloïde comprend :

(a) 0,2 à 5 parties en poids d'un ester d'acide gras de sorbitan éthoxylé,
(b) 20 à 50 parties en poids d'un hydrocolloïde,
(c) 32 à 120 parties en poids d'une matrice adhésive constituée à partir d'un ou plusieurs polymères choisis parmi les copolymères séquencés poly(styrène-oléfine-styrène), les polyisobutylènes de bas poids moléculaire, les polyisobutylène de haut poids moléculaire, et un ou plusieurs composés choisis parmi les résines poisseuses dites tackifiantes, les plastifiants, les polybutènes, les antioxydants, les copolymères d'éthylène et d'acétate de vinyle, les caoutchoucs butyle et les copolymères blocs éthylène-propylène ; et,
(d) 0 à 15 parties en poids d'un polymère acrylate ayant une température de transition vitreuse inférieure à - 20 °C.

Par ester d'acide gras de sorbitan éthoxylé on entend désigner ici les esters d'acide gras (notamment les monoesters, triesters ou leurs mélanges) de sorbitan éthoxylé où chaque partie acide gras de l'ester contient de 8 à 22 atomes de carbone et constitue une chaîne linéaire ou ramifiée, de préférence une chaîne linéaire, saturée ou possèdant un ou plusieurs sites d'insaturation oléfinique

Dans le cadre de la présente invention, on préférera en particulier les esters d'acides gras de sorbitan éthoxylé que l'on désigne sous le terme de polysorbate et qui correspondent aux monoesters et triesters d'acide gras de sorbitan éthoxylé tels que représentés respectivement par les formules I et II ci-dessous dans lesquelles la somme w+ x+y+z peut prendre les valeurs 20, 5 ou 4 et R représente la partie carbonée de l'acide gras qui contient de 7 à 21 atomes de carbone.

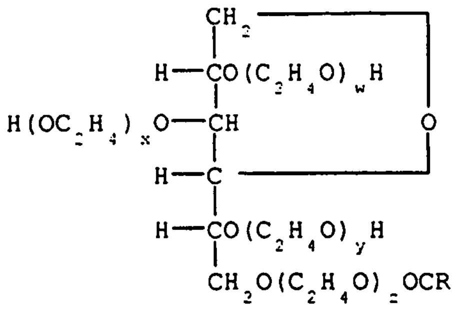

(I)

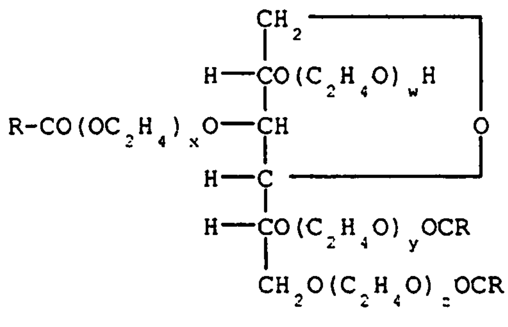

(II)

On préférera ainsi parmi les monoesters de sorbitan éthoxylé de formule I ceux pour lesquels la somme w+x+y+z=20 comme par exemple le monolaurate de sorbitan ethoxylé (appelé aussi polysorbate 20) le monopalmitate de sorbitan éthoxylé (appelé aussi polysorbate 40), le monostéarate de sorbitan éthoxylé (appelé aussi polysorbate 60), le monooléate de sorbitan éthoxylé (appelé aussi polysorbate 80), le monoisostéarate de sorbitan éthoxylé (appelé aussi polysorbate 120). Parmi les monoesters de formule I pour lesquels w+x+y+z = 4 on préférera le monolaurate de sorbitan éthoxylé (appelé aussi polysorbate 21) et le monostéarate de sorbitan éthoxylé (appelé aussi polysorbate 61) et enfin parmi ceux pour lesquels w+x+y+z=5, le monoléate de sorbitan éthoxylé (appelé aussi polysorbate 81).

De même on préférera parmi les triesters de sorbitan éthoxylé de formule II ceux pour lesquels la somme w+x+y+z=20 comme par exemple le tristéarate de sorbitan éthoxylé (appelé aussi polysorbate 65) et le trioléate de sorbitan éthoxylé (appelé aussi polysorbate 85).

Dans le cadre de la présente invention, on préférera tout particulièrement le polysorbate 80 comme par exemple les produits commercialisés par la société SEPPIC sous les dénominations respectives MONTANOX® 80 et MONTANOX® 80VG (lequel a une composition identique au précédent, mais dans ce cas la partie acide gras est d'origine végétale).

Selon un second aspect, la présente invention a pour objet l'utilisation de ces masses adhésives hydrocolloïdes pour la réalisation de pansements notamment pour le traitement de l'ampoule, des lésions dermo-épidermiques superficielles, profondes, chroniques ou aiguës et le traitement des plaies exsudatives et brûlures.

Les matrices adhésives utilisables dans la réalisation des masses adhésives hydrocolloïdes sont celles couramment employées par l'homme de métier. Elles sont formées d'au moins un élastomère choisi parmi les polymères tels que les polyisobutylènes ou des copolymères séquencés poly(styrène-oléfine-styrène) comme par exemple poly (styrène-isoprène-styrène), poly(styrène-butadiène-styrène) ou poly(styrène-éthylène-butylène-styrène) associé ou non à des antioxydants, des agents permetttant d'améliorer l'adhésion tels que des résines poisseuses dites « tackifiantes, des plastifiants tels que les polybutènes ou les huiles plastifiantes, ou à des agents permettant d'améliorer la cohésion tels que des caoutchoucs butyle, etc...

De telles compositions sont ainsi définies dans « Advances in Pressure Sensitive Adhesive Technology-2 » édité par Donatas Satas en avril 95 dans le chapitre 7 « Wound Dressings » page 158 à 171.

L'on pourra aussi se reporter à cet égard aux documents de l'état de la technique mentionnés précédemment pour les définitions de l'ensemble des composés utilisés dans ces formulations et leurs proportions respectives dans la matrice adhésive.

Ainsi dans le cas d'une matrice adhésive à base d'élastomère de polyisobutylène, on utilisera des polyisobutylènes de bas poids moléculaire, de l'ordre de 40 000 à 80 000 daltons tels que les composés commercialisés sous la dénomination VISTANEX® par la société EXXON CHEMICAL ou sous la dénomination OPPANOL® par la société BASF.

On préférera en particulier les produits commercialisés sous les dénominations VISTANEX®LM-MS, VISTANEX®LM-MH, OPPANOL®B12 et OPPANOL®B15.

Ces derniers pourront être utilisés seul ou en mélange.

Ces polyisobutylènes pourront si nécessaire être associés à des composés supplémentaires pour améliorer les propriétés d'élasticité, de résistance ou de cohésion tels que des polyisobutylènes de haut poids moléculaire, de l'ordre de 400 000 à 2 000 000 daltons comme par exemple les produits commercialisés par la société EXXON CHEMICAL sous les dénominations VISTANEX®L-80 ou VISTANEX®L-100, des copolymères éthylène-acétate de vinyle, comme par exemple ceux commercialisés sous la dénomination ELVAX® par la société DUPONT ou sous les dénominations ULTRATHENE® et VYNATHENE® par la société U.S.I. Chemicals, des copolymères blocs éthylène-propylène, comme par exemple ceux commercialisés par la société DUPONT sous la dénomination NORDEL®, ou des caoutchoucs butyle comme par exemple ceux commercialisés sous les dénominations GRADE®065 ou GRADE®067 par la société EXXON

CHEMICAL.

On peut aussi incorporer dans ces matrices adhésives des plastifiants tels que des polybutènes comme par exemple ceux commercialisés par la société BP CHEMICALS sous la dénomination NAPVIS®10.

Les formulations de telles matrices adhésives seront réalisées de façon classique par l'homme du métier de manière à obtenir les propriétés adhésives et mécaniques souhaitées en adaptant la quantité de chaque composé.

On pourra de même ajouter à ces matrices adhésives à base de polyisobutylènes des copolymères séquencés du type poly(styrène-oléfine-styrène). On utilisera alors de préférence des poly(styrène-isoprène-styrène), comme par exemple les produits commercialisés sous les dénominations KRATON®D-1107 ou KRATON® 1161 par la société SHELL CHEMICALS ou le produit commercialisé sous la dénomination VECTOR® 4113 par la société EXXON CHEMICAL, ou des poly(styrène-butadiène-styrène) comme par exemple le produit commercialisé sous la dénomination KRATON®D-1102 par la société SHELL CHEMICALS.

De telles formulations de matrices adhésives sont par exemple décrites dans la demande de brevet EP-A-130061.

De même on pourra utiliser des matrices adhésives uniquement à base d'élastomère du type copolymères séquencés poly(styrène-oléfine-styrène), en particulier à base de poly(styrène-isoprène-styrène) ou poly(styrène-éthylène-butylène-styrène) que l'on associe pour obtenir les propriétés adhésives et cohésives souhaitées à des plastifiants, résines « tackifiantes », antioxydants etc...

De telles formulations de matrices adhésives sont aussi parfaitement connues de l'homme de l'art.

Les copolymères séquencés du type styrène-oléfine-styrène qui sont susceptibles d'être utilisés dans le cadre de la préparation de ces matrices adhésives sont ceux habituellement utilisés par l'homme de l'art et l'on pourra se reporter à cet égard au document de l'état de la technique mentionné précédemment.

Les séquences oléfines de ces copolymères peuvent être constituées d'unités isoprène, butadiène ou éthylène-butylène, éthylène-propylène et leurs mélanges.

Dans le cadre de la présente invention, les copolymères triblocs poly(styrène-isoprène-styrène) sont préférés.

Par copolymère tribloc du type poly(styrène-isoprène-styrène) [en abrégé : poly(SIS)] on entend ici un matériau poly(SIS) ayant une teneur en styrène comprise entre 14 et 52 % en poids par rapport au poids dudit poly(SIS). Cette expression couvre aussi des poly(SIS) contenant un mélange de copolymères tribloc poly(SIS) et de copolymères dibloc du type poly(styrène-isoprène).

De tels produits bien connus de l'homme de l'art sont par exemple commercialisés par les sociétés SHELL et EXXON CHEMICAL respectivement sous les dénominations KRATON®D et VECTOR®.

Dans le cadre de la présente invention on préfère les copolymères tribloc ayant une teneur en styrène comprise entre 14 et 30 % en poids par rapport au poids dudit poly(SIS). On préfèrera en particulier les produits commercialisés respectivement sous les dénominations VECTOR®4114 par la société EXXON CHEMICAL et KRATON®D-1111CS par la société SHELL CHEMICALS.

Parmi les résines tackifiantes qui conviennent pour réaliser ces matrices adhésives, on peut mentionner les résines généralement employées dans le domaine des adhésifs par l'homme de l'art telles que les résines polyterpènes ou terpènes modifiées, les résines de colophane hydrogénée, les résines de colophane polymérisée, les résines d'esters de colophane, les résines hydrocarbonées, les mélanges de résines aromatiques et aliphatiques etc... On préfèrera en particulier dans le cadre de la présente invention une résine de synthèse formée de copolymères en $C_5/C_9$ commercialisée par la société GOOD YEAR sous la dénomination WINGTACK®86.

Par agents antioxydants on entend désigner ici les composés couramment employés par l'homme de l'art pour assurer la stabilisé vis-à-vis de l'oxygène, la chaleur, l'ozone et les rayonnements ultra violets des composés utilisés dans la formulation des matrices, en particulier les résines tackifiante et les copolymères séquencés. On peut utiliser un ou plusieurs de ces agents antioxydants en association.

On peut citer comme agents antioxydants appropriés les antioxydants phénoliques comme par exemple les produits commercialisés par la société CIBA-GEIGY sous les dénominations IRGANOX®1010, IRGANOX®565, IRGANOX® 1076 et des antioxydants soufrés comme par exemple le dibutyldithiocarbamate de zinc commercialisé par la société AKZO sous la dénomination PERKACIT®ZDBC.

Dans le cadre de la présente invention, on préfèrera l'association de l'IRGANOX® 1010 et du PERKACIT®ZDBC.

Tout type de plastifiant habituellement utilisé par l'homme de l'art pour la préparation de matrice adhésive à base de copolymère séquencé styrène-oléfine-styrène peut être utilisé dans le cadre de la présente invention. On préfèrera cependant utiliser des huiles plastifiantes.

Par huile plastifiante on entend désigner ici les huiles minérales ou végétales couramment employées par l'homme de l'art pour plastifier les copolymères séquencés du type styrène-oléfine-styrène utilisés dans la composition des matrices adhésives employées dans les masses adhésives hydrocolloïdes.

Les huiles minérales généralement utilisées sont des mélanges de composés de nature paraffinique, naphténique ou aromatique dans des proportions variables.

On peut citer ainsi comme exemple d'huiles plastifiantes les produits commercialisés par la société SHELL

sous la dénomination ONDINA® et RISELLA® pour les mélanges à base de composés naphténiques et paraffiniques ou sous la dénomination CATENEX® pour les mélanges à base de composés naphténiques, aromatiques et paraffiniques.

Dans le cadre de la présente invention, on préférera en particulier l'huile plastifiante minérale commercialisée sous la dénomination ONDINA®68.

Dans le cadre de la réalisation des masses adhésives hydrocolloïdes selon l'invention, on entend ici par hydrocolloïde les composés couramment utilisés par l'homme de l'art, connus pour leur aptitude à absorber les liquides hydrophiles, en particulier l'eau et les transporter rapidement. Comme hydrocolloïdes appropriés, on peut citer par exemple l'alcool polyvinylique, la gélatine, la pectine, les alginates de sodium, les gommes végétales naturelles telles que la gomme de caroube, la gomme de Karaya, la gomme guar, la gomme arabique..., les dérivés de cellulose tels que les hydroxyéthylcelluloses, les hydroxypropylcelluloses, les carboxyméthylcelluloses et leurs sels de métal alcalin, tel le sodium ou le calcium. On pourra utiliser ces hydrocolloïdes seuls ou en association.

Dans le cadre de la présente invention on préférera les sels de métal alcalin de carboxyméthylcellulose; en particulier la carboxyméthylcellulose de sodium.

Selon un mode de réalisation actuellement préféré, la masse adhésive hydrocolloïde contient en outre un polymère acrylate ayant une température de transition vitreuse (Tg) inférieure à - 20 °C.

De tels composés d'acrylates sont des copolymères formés à partir

- soit d'au moins un monomère, choisi parmi l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle, associé avec l'acide acrylique;
- soit d'au moins un monomère, choisi parmi l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle.

Les pourcentages ou proportions respectifs de ces différents monomères sont ajustés de façon à obtenir un copolymère ayant la température de transition vitreuse souhaitée c'est à dire inférieure à -20 °C.

Dans le cadre de la présente invention, on utilisera de préférence un copolymère contenant au moins un monomère, choisi parmi l'acrylate de n-butyle, l'acrylate de 2-éthylhexyle et l'acrylate d'isooctyle, copolymérisé avec l'acide acrylique.

On préférera tout particulièrement les copolymères contenant de 1 à 20 %, de préférence 1 à 10 %, en poids d'acide acrylique, exprimé par rapport au poids total de l'ensemble des monomères.

De tels composés d'acrylates peuvent aussi être des homopolymères dont le monomère constitutif est choisi dans l'ensemble constitué par les esters alkylés de l'acide acrylique dans lesquels le groupe alkyle de l'ester est soit un groupe alkyle linéaire qui comprend 2 à 12 atomes de carbone soit un groupe isobutyle, 2-éthylhexyle ou isooctyle.

Parmi ces homopolymères on préférera dans le cadre de la présente invention, le polyacrylate de n-butyle.

Selon une caractéristique particulière de l'invention, on choisira les produits bien connus de l'homme de l'art pour être utilisables dans un procédé d'enduction sans solvant connu sous le nom de « hot-melt ».

On peut ainsi citer par exemple les produits commercialisés par la société BASF sous les dénominations suivantes:

- ACRONAL®A150F (homopolymère d'acrylate de n-butyle qui possède une température de transition vitreuse de -41 °C),
- ACRONAL®DS3429 (copolymère d'acrylate de n-butyle, d'acrylate de 2-éthylhexyle et d'acide acrylique qui possède une température de transition vitreuse de -31 °C) et ACRONAL®DS3458 (copolymère d'acrylate de n-butyle et d'acide acrylique qui possède une température de transition vitreuse de -39 °C.)

On peut citer également le produit commercialisé par la société MONSANTO sous la dénomination :

- MODAFLOW® (copolymère d'acrylate d'éthyle et d'acrylate de 2-éthylhexyle).

Dans le cadre de la présente invention, on préférera tout particulièrement le polymère acrylate commercialisé sous la dénomination ACRONAL®DS3458.

Selon un mode de réalisation particulièrement préféré de la présente invention, pour les matrices adhésives uniquement à base de copolymères séquencés poly(styrène-oléfine-styrène) on préconise une masse adhésive hydrocolloïde qui comprend :

a) 10 à 35 parties en poids d'un copolymère tribloc poly(styrène-isoprène-styrène),
b) 20 à 50 parties en poids d'une résine tackifiante,
c) 0,5 à 15 parties en poids d'un polymère acrylate ayant une température de transition vitreuse inférieure à - 20 °C,
d) 2 à 25 parties en poids d'une huile plastifiante,
e) 20 à 50 parties en poids d'un hydrocolloïde,
f) 0,1 à 2 parties en poids d'au moins un agent antioxydant,
g) 0,2 à 5 parties en poids de monooléate de sorbitan éthoxylé.

Selon un mode de réalisation encore préféré, cette masse adhésive hydrophile comprend, pour un total de 100 parties en poids :

a) 18 à 22, et de préférence 17,7 parties en poids d'un copolymère tribloc poly(styrène-isoprène-styrène),
b) 20 à 35, et de préférence 26,5 parties en poids d'une résine tackifiante,
c) 3 à 8, et de préférence 6,5 parties en poids d'un copolymère d'acrylate de n-butyle et d'acide acrylique qui présente une température de transition vitreuse de - 39 °C.
d) 10 à 20, et de préférence 12,4 parties en poids d'une huile plastifiante minérale,
e) 25 à 40, et de préférence 35,7 parties en poids de carboxyméthylcellulose de sodium,
f) 0,3 à 0,8, et de préférence 0,75 parties en poids d'un agent antioxydant phénolique,
0,3 à 0,8, et de préférence 0,35 parties en poids d'un agent antioxydant soufré, le dibutyldithiocarbamate de zinc, et,
g) 0,2 à 3 et de préférence 0,5 parties en poids de polysorbate 80.

De même, selon un mode de réalisation particulièrement préféré de l'invention, pour les matrices adhésives à base de polyisobutylène, on préconise une masse adhésive hydrocolloïde qui comprend :

a) 5 à 20 parties en poids d'un copolymère (styrène-isoprène-styrène)
b) 25 à 50 parties en poids d'un polyisobutylène de bas poids moléculaire,
c) 2 à 20 parties en poids d'un polybutène,
d) 20 à 50 parties en poids d'un hydrocolloïde,
e) 0,2 à 5 parties en poids d'un monooléate de sorbitan éthoxylé,
f) 0,5 à 15 parties en poids d'un polymère acrylate ayant une température de transition vitreuse inférieure à - 20 °C
g) 0,1 à 2 parties en poids d'au moins un antioxydant.

La masse adhésive hydrocolloïde selon l'invention est utile pour toute application à des fins médicales dans laquelle il est nécessaire d'absorber dès les premières heures des fluides ou des liquides. On peut ainsi citer la réalisation de pansements et de bandages pour le traitement de l'ampoule, des lésions dermo-épidermiques superficielles, profondes, chroniques ou aiguës, des plaies exsudatives, des brûlures ainsi que la réalisation de joints adhésifs employés en ostomies.

Dans le cadre de ces applications divers produits à caractère dermatologique, cosmétologique, ou thérapeutique peuvent être ajoutés dans la formulation de la masse adhésive hydrocolloïde comme par exemple des antifongiques, des anti-microbiens ou antibactériens tels que la sulfadiazine argentique, des régulateurs de pH, des accélérateurs de cicatrisation, des vitamines, des extraits végétaux, des oligo-éléments, des anesthésiques locaux, des piégeurs d'odeur, du menthol, du salicylate de méthyle, des hormones, des anti-inflammatoires, etc...

Dans le cadre de la réalisation d'un pansement pour le traitement de l'ampoule ou le traitement ou la protection des plaies, de différentes catégories de lésions dermo-épidermiques, brûlures et escarres, on réalise une enduction de la masse adhésive hydrocolloïde selon l'invention sur un support adéquat au grammage souhaité, selon les techniques connues de l'homme de l'art, suivant un procédé en phase solvant ou de préférence suivant un procédé hot-melt, c'est à dire sans solvant, à une température comprise entre 110 et 160 °C.

Le choix du support est réalisé en fonction des propriétés requises (étanchéité, élasticité etc...) suivant le type de pansement et l'application recherchée.

Il peut se présenter sous forme d'un film d'une épaisseur variable de 5 à 150 μm ou d'un non-tissé ou d'une mousse ayant une épaisseur de 10 à 500 μm. Ces supports à base de matériaux synthétiques ou naturels sont ceux généralement utilisés par l'homme de l'art dans le domaine des pansements et des applications médicales visées ci-dessus.

On peut citer ainsi des mousses en polyéthylène, en polyuréthanne, en PVC, des non-tissés en polypropylène, polyamide, polyester, éthylcellulose etc... On préférera cependant utiliser des films en tant que support et notamment des films en polyuréthanne, des films en polyéthylène basse densité comme par exemple ceux commercialisés par la

société SOPAL, des films à base de copolymère thermoplastique de polyéther-polyester comme par exemple les produits commercialisés sous la marque Hytrel® par la société DUPONT DE NEMOURS ou des complexes réalisés à base de polyuréthanne et d'un non-tissé.

On préférera dans le cadre de la présente invention des films en polyuréthanne comme par exemple les produits commercialisés par la société Smith et Nephew sous la référence LASSO ou des films en polyuréthanne réalisés à partir du polyuréthanne commercialisé sous la dénomination UCECOAT® par la société UCB ou sous la dénomination ESTANE® par la société BF GOODRICH.

Les pansements réalisés à partir de la masse adhésive hydrocolloïde selon l'invention peuvent se présenter sous n'importe quelle forme géométrique, carrée, rectangulaire, circulaire ou ovale. De même leur taille peut être quelconque en fonction de la surface de la partie à traiter ou protéger.

De façon pratique, la surface de la masse adhésive qui n'est pas liée au support, pourra être recouverte d'une couche ou pellicule de protection pelable avant utilisation du pansement. L'ensemble ainsi formé pourra être lui-même emballé dans une protection étanche réalisée par exemple au moyen de complexes polyéthylène-aluminium ou dans des blisters.

Les avantages, caractéristiques et applications de l'invention seront mieux compris à la lecture de la description qui va suivre d'exemples de réalisation et d'essais comparatifs.

Bien entendu l'ensemble de ces éléments n'est nullement limitatif mais est donné à titre d'illustration.

Par commodité, dans ce qui suit les abréviations suivantes ont été utilisées :

SIS : copolymère tribloc poly(styrène-isoprène-styrène)

Exemple 1

Dans un malaxeur à bras en Z, à une température de l'ordre de 130 °C on introduit successivement 12,4 kg de ONDINA®68 (huile minérale commercialisée par la société SHELL), 17,7 kg de VECTOR®4114 (copolymère SIS commercialisé par la société DEXCO), 0,35 kg de PERKACIT®ZDBC (dibutyl dithiocarbamate de zinc, antioxydant commercialisé par la société AKZO) et 0,4 kg d'IRGANOX®1010 (antioxydant commercialisé par la société CIBA-GEIGY). On malaxe le mélange obtenu entre 120 et 140 °C durant environ 30 minutes. On ajoute ensuite 6,5 kg d'ACRONAL®DS 3458 (copolymère de butylacrylate et d'acide acrylique commercialisé par la société BASF) et on malaxe le mélange obtenu toujours aux alentours de 130 °C durant 10 minutes. On ajoute alors 26,5 kg de WINGTACK®86 (résine tackifiante commercialisée par la société GOOD YEAR) et on malaxe toujours aux alentours de 130 °C durant 20 minutes. On introduit alors 0,45 kg de MONTANOX®80VG (polysorbate 80) et on malaxe 15 minutes supplémentaires à environ 130 °C. On introduit finalement 35,7 kg de BLANOSE®7H4XF (carboxyméthylcellulose de sodium commercialisé par la société AQUALON) et on malaxe toujours à environ 130 °C durant 30 minutes supplémentaires.

On enduit le mélange ainsi obtenu sur une pellicule de papier siliconé à raison de 1000 $g/m^2$ à une température comprise entre 120 et 160 °C. On transfère l'enduction ainsi réalisée sur un support final, de 30 μm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

Exemple 2

On procède de façon analogue à l'exemple 1 mais dans ce cas on utilise une carboxyméthylcellulose de sodium de granulométrie différente. On remplace donc ici 35,7 kg de BLANOSE®7H4XF par la même quantité de BLANOSE®7H3XF (produit commercialisé par la société AQUALON). De plus, dans cet exemple, on enduit sur une pellicule de papier siliconé à raison de 600 $g/m^2$.

Exemple 3

On procède de façon analogue à l'exemple 1 mais on utilise dans ce cas une autre carboxyméthylcellulose de sodium aux propriétés d'absorption différentes. On remplace donc ici 35,7 kg de BLANOSE®7H4XF par la même quantité d'AQUASORB®A500 (produit commercialisé par la société AQUALON). De plus, dans cet exemple, on enduit sur une pellicule de papier siliconé à raison de 400 $g/m^2$.

Exemple 4

Dans un malaxeur à bras en Z, à une température de l'ordre de 130 °C on introduit successivement 13,8 kg de ONDINA®68, 19,7 kg de VECTOR®4114, 0.4 kg de PERKACIT®ZDBC et 0,4 kg d'IRGANOX®1010. On malaxe le

mélange obtenu toujours aux environs de 130 °C durant environ 30 minutes. On ajoute ensuite 29,5 kg de WINGTACK®86 et on malaxe toujours à environ 130 °C durant 20 minutes. On introduit alors 0,5 kg de MONTANOX®80VG et on malaxe 15 minutes supplémentaires à environ 130 °C. On introduit finalement 35,7 kg de AQUASORB®A500 et on malaxe toujours à environ 130 °C durant 30 minutes supplémentaires. On enduit le mélange ainsi obtenu sur une pellicule de papier siliconé à raison de 1000 $g/m^2$ à une température comprise entre 120 et 160 °C. On transfère l'enduction ainsi réalisée sur un support final, de 30 μm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

Exemple 5

On procède de façon analogue à l'exemple 4 mais dans ce cas on utilise une autre carboxyméthylcellulose de sodium. On remplace donc ici 35,7 kg de AQUASORB®A500 par la même quantité de BLANOSE®7H4XF. De plus, dans cet exemple, on enduit sur une pellicule de papier siliconé à raison de 600 $g/m^2$.

Exemple 6

On procède de façon analogue à l'exemple 5 mais dans ce cas on utilise une carboxyméthylcellulose de sodium de granulométrie différente. On remplace donc ici 35.7 g de BLANOSE®7H4XF par la même quantité de BLANOSE®7H3XF. De plus, dans cet exemple, on enduit sur une pellicule de papier siliconé à raison de 400 $g/m^2$.

Exemple 7

Dans un malaxeur à bras en Z, à une température de l'ordre de 130 °C on introduit successivement 15,3 kg de VECTOR®4113 (copolymère SIS commercialisé par la société DEXCO), 39,4 kg de VISTANEX®LM-MH (polymère PIB de bas poids moléculaire commercialisé par la société EXXON CHEMICAL), 0.2 kg d'IRGANOX®1010 et 8,1 kg de NAPVIS®10 (polybutène commercialisé par la société BP CHEMICALS). On malaxe le mélange obtenu à environ 140 °C pendant environ 40 minutes. On ajoute alors 6,5 kg de d'ACRONAL®DS 3458 et on malaxe le mélange obtenu toujours vers 140 °C durant environ 10 minutes. On ajoute ensuite 0,5 kg de MONTANOX®80VG et on malaxe toujours à environ 140 °C durant 10 minutes. On introduit finalement 30 kg de BLANOSE®7H4XF et on malaxe aux alentours de 140 °C durant environ 20 minutes supplémentaires.

On enduit le mélange ainsi obtenu sur une pellicule de papier siliconé à raison de 1000 $g/m^2$ à une température comprise entre 120 et 160 °C. On transfère l'enduction ainsi réalisée sur un support final de 30 μm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

Exemple 8

Dans un malaxeur à bras en Z, à une température de l'ordre de 140 °C on introduit successivement 13,8 kg de ONDINA®68, 15,8 kg de VECTOR®4114, 3,9 kg de VISTANEX®LM-MH, 0,4 kg de PERKACIT®ZDBC et 0,4 kg d'IRGANOX®1010. On malaxe le mélange obtenu à environ 140 °C pendant environ 30 minutes. On introduit alors 29,5 kg de WINGTACK®86 et on malaxe toujours vers 140 °C durant environ 35 minutes supplémentaires. On ajoute alors 0,5 kg de MONTANOX®65 (polysorbate 65) et on malaxe à 140 °C environ 40 minutes. On introduit finalement 35,7 kg de BLANOSE®7H4XF et on malaxe aux alentours de 140 °C durant environ 45 minutes. On enduit le mélange ainsi obtenu sur une pellicule de papier siliconé à raison de 1000 $g/m^2$ à une température comprise entre 120 et 160 °C. On transfère l'enduction ainsi réalisée sur un support final de 30 μm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

Exemple 9

On procède de façon analogue à l'exemple 1 mais dans ce cas on utilise 0,35 kg d'IRGANOX®1010 et 0,5 kg d'un autre ester de sorbitan éthoxylé le MONTANOX®65 (polysorbate 65).

Exemple 10

On procède de façon analogue à l'exemple 9 mais on utilise dans ce cas le MONTANOX®60 (polysorbate 60).

Exemple 11

On procède de façon analogue à l'exemple 9 mais on utilise dans ce cas le TWEEN®81 (polysorbate 81).

Exemple 12

On procède de façon analogue à l'exemple 1 mais dans ce cas on utilise 14,1 kg de CATENEX®N945 (huile minérale commercialisée par la société SHELL), 19,6 kg de VECTOR®4114, 0,4 kg de PERKACIT®ZDBC, 0,4 kg d'IRGANOX®1010, 4,9 kg d'ACRONAL®DS3458, 29,7 kg de WINGTACK®86, 1 kg de MONTANOX®80VG et 30 kg de BLANOSE®7H4XF. On enduit de la même façon à raison de 6000 g/m$^2$ et on transfère l'enduction ainsi réalisée sur un support final de 30 μm d'épaisseur, en polyuréthanne commercialisé sous la dénomination LASSO 687 par la société Smith et Nephew.

Exemple 13

On procède de façon identique à l'exemple 12 mais dans ce cas on utilise la même quantité 1 kg d'un autre ester de sorbitan éthoxylé le MONTANOX®65 (polysorbate 65).

Exemple 14

On procède de façon identique à l'exemple 1 mais dans ce cas on utilise une quantité plus faible 0,1 kg de MONTANOX®80VG. On utilise donc 12,4 kg de ONDINA®68, 17,8 kg de VECTOR®4114, 0,35 kg de PERKACIT®ZDBC, 0,35 kg d'IRGANOX®1010, 6,6 kg d'ACRONAL®DS 3458, 26,6 kg de WINGTACK®86, 0,1 kg de MONTANOX®80VG et 35,8 kg de BLANOSE®7H4XF.

Exemple comparatif 1

Dans un malaxeur à bras en Z, à une température de l'ordre de 130 °C on introduit successivement 12.5 kg de ONDINA®68, 17,8 kg de VECTOR®4114, 0.35 kg de PERKACIT®ZDBC et 0.35 kg d'IRGANOX®1010. On malaxe le mélange obtenu à 130 °C durant 35 minutes. On ajoute ensuit 6,5 kg d'ACRONAL®DS 3458 et on malaxe le mélange obtenu toujours à 130 °C durant 10 minutes. On introduit alors 26,7 kg de WINGTACK®86 et on malaxe toujours à 130 °C durant 20 minutes supplémentaires. On introduit finalement 35,7 kg de BLANOSE®7H4XF et on malaxe encore durant 25 minutes. On enduit le mélange ainsi obtenu sur une pellicule de papier siliconé à raison de 1000 g/m$^2$ à une température comprise entre 120 et 160 °C. On transfère l'enduction ainsi réalisée sur un support final, de 30 μm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

Exemple comparatif 2

On procède de façon analogue à l'exemple comparatif 1 mais on utilise dans ce cas une carboxyméthylcellulose de sodium de granulométrie différente. On remplace donc ici 35,7 kg de BLANOSE®7H4XF par la même quantité de BLANOSE®7H3XF. De plus, dans cet exemple, on enduit sur une pellicule de papier siliconé à raison de 600 g/m$^2$.

Exemple comparatif 3

On procède de façon analogue à l'exemple comparatif 1 mais dans ce cas on utilise une autre carboxyméthylcellulose de sodium aux propriétés d'absorption différentes. On remplace donc ici 35,7 kg de BLANOSE®7H4XF par la même quantité de AQUASORB®A500. De plus, dans cet exemple, on enduit sur une pellicule de papier siliconé à raison de 400 g/m$^2$.

Exemple comparatif 4

Dans un malaxeur à bras en Z, à une température de l'ordre de 130 °C on introduit successivement 13,9 kg de ONDINA®68, 19,8 kg de VECTOR®4114, 0,4 kg de PERKACIT®ZDBC ET 0,4 kg d'IRGANOX®1010. On malaxe le mélange obtenu à 130 °C durant 35 minutes. On introduit alors 29,8 kg de WINGTACK®86 et on malaxe toujours à 130 °C durant 20 minutes supplémentaires. On ajoute finalement 35,7 kg d'AQUASORB®A500 et on malaxe encore durant 25 minutes. On enduit le mélange ainsi obtenu sur une pellicule de papier siliconé à raison de 1000 g/m$^2$ à une température

comprise entre 120 et 160 °C. On transfère l'enduction ainsi réalisée sur un support final de 30 µm d'épaisseur, à base de polyuréthanne UCECOAT® identique à celui de l'exemple 4. On découpe alors des formes aux dimensions appropriées que l'on conditionne dans des sachets thermosoudables ou des blisters.

Exemple comparatif 5

On procède de façon analogue à l'exemple comparatif 4 mais on emploie dans ce cas une autre carboxyméthylcellulose de sodium. On remplace l'AQUASORB®A500 par la même quantité de BLANOSE®7H4XF. On enduit dans ce cas à 600 g/m$^2$.

Exemple comparatif 6

On procède de façon analogue à l'exemple comparatif 5 mais on emploie dans ce cas une carboxyméthylcellulose de sodium de granulométrie différente. On remplace le BLANOSE®7H4XF par la même quantité de BLANOSE®7H3XF. De plus on enduit dans ce cas à 400 g/m$^2$.

Exemple comparatif 7

Dans un malaxeur à bras en Z, à une température de l'ordre de 130 °C on introduit successivement 15,4 kg de VECTOR®4113, 39,7 kg de VISTANEX®LM-MH, 0,2 kg d'IRGANOX®1010, et 8,2 kg de NAPVIS®10, on malaxe le mélange obtenu à environ 140 °C pendant environ 40 minutes. On ajoute alors 6,5 kg d'ACRONAL®DS 3458 et on malaxe le mélange obtenu toujours vers 140 °C durant environ 10 minutes. On introduit finalement 30 kg de BLANOSE®7H4XF et on malaxe aux alentours de 140 °C durant environ 20 minutes supplémentaires. On enduit le mélange ainsi obtenu sur une pellicule de papier siliconé à raison de 1000 g/m$^2$ à une température comprise entre 120 et 160 °C. On transfère l'enduction ainsi réalisée sur un support final, de 30 µm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

Exemple comparatif 8

Dans un malaxeur à bras en Z, à une température de l'ordre de 140 °C on introduit sucessivement 13,95 kg de ONDINA®68, 15,8 kg de VECTOR®4114, 3,95 kg de VISTANEX®LM-MH, 0,4 kg de PERKACIT®ZDBC et 0,4 kg d'IRGANOX®1010. On malaxe le mélange obtenu à environ 140 °C pendant environ 30 minutes. On introduit alors 29,8 kg de WINGTACK®86 et on malaxe toujours à 140 °C durant environ 35 minutes supplémentaires. On introduit finalement 35,7 kg de BLANOSE®7H4XF et on malaxe aux alentours de 140 °C durant encore 45 minutes. On enduit le mélange ainsi obtenu sur une pellicule de papier siliconé à raison de 1000 g/m$^2$ à une température comprise entre 120 et 160 °C. On transfère l'enduction ainsi réalisée sur un support final de 30 µm d'épaisseur, en polyuréthanne (réalisé à partir de polyuréthanne commercialisé sous la dénomination UCECOAT® par la société UCB). On découpe alors des formes aux dimensions appropriées que l'on conditionne dans des sachets thermosoudables ou des blisters.

Exemple comparatif 9

On procède de façon analogue à l'exemple comparatif 1 mais on utilise dans ce cas des quantités différentes en particulier de BLANOSE®7H4XF et d'ACRONAL®DS 3458, une autre huile minérale le CATENEX®N945 et un autre support. On emploie donc 14,3 kg de CATENEX®N945, 19,8 kg de VECTOR®4114, 0,4 kg de PERKACIT®ZDBC, 0,4 kg d'IRGANOX®1010, 4,9 kg d'ACRONAL®DS 3458, 30,2 kg de WINGTACKC®86 et 30 kg de BLANOSE®7H4XF. On enduit de la même façon entre 120 et 160 °C le mélange obtenu sur une pellicule de papier siliconé à raison de 600 g/m$^2$. On transfère l'enduction ainsi réalisée sur un support final, de 30 µm d'épaisseur, en polyuréthanne commercialisé sous la dénomination LASSO 687 par la société Smith et Nephew.

Essais

Afin de mettre en évidence l'augmentation de la capacité d'absorption des masses adhésives hydrocolloïdes selon l'invention dès la première heure on a réalisé des mesures d'absorption avec différents échantillons obtenus à partir des exemples 1 à 14. On a ainsi pour chaque exemple n la valeur d'absorption à une heure A(EXn).
Dans un but comparatif on a déterminé de la même façon l'absorption de masses adhésives hydrocolloïdes identiques mais sans incorporation d'un ester d'acide gras de sorbitan éthoxylé à partir d'échantillons correspondants aux exemples comparatifs 1 à 9. On a ainsi pour chaque exemple comparatif n la valeur d'absorption à une heure A(ECn).

Ces mesures ont été effectuées suivant le protocole suivant :

On utilise un échantillon, réalisé comme décrit dans les exemples 1 à 14 et les exemples comparatifs 1 à 9, formé du support final, de la masse adhésive hydrocolloïde et de la pellicule de papier siliconé qui sert de protecteur pelable, que l'on découpe de façon à obtenir un ruban adhésif. Pour réaliser la mesure on utilise une cellule de mesure constituée d'un cylindre en aluminium sur lequel on dépose un échantillon de ruban adhésif à tester et sur lequel on vient ensuite fixer un support qui permet de bien solidariser l'ensemble cylindre-échantillon. La partie périphérique de ce support comporte un joint siliconé sur lequel s'adhésive par pression la section périphérique de l'échantillon.

La mesure d'absorption s'effectue par différences de pesées de l'ensemble support-ruban adhésif-cylindre avant et après mise en contact de l'échantillon avec un liquide de référence durant une durée déterminée, ici une heure.

Dans les tests suivants le liquide de référence est une solution de dextran D4876 (commercialisé par la société Sigma) à 60 g par litres dans une solution de chlorure de sodium 0,15 molaire.

Les mesures sont réalisées suivant les étapes suivantes :

- on découpe un échantillon (ici par exemple de 16 $cm^2$) du ruban adhésif à tester et on enlève le film protecteur.
- on incorpore l'échantillon dans la cellule de mesure comme décritci-dessus
- l'ensemble ainsi obtenu est pesé ; soit $P_0$ le poids obtenu
- on introduit alors 20 ml du liquide de référence précédemment préparé dans le cylindre
- on laisse l'ensemble en contact à 23 °C pendant 1 heure
- à l'issue d'une heure on pèse à nouveau, après élimination de la solution non absorbée, l'ensemble support-échantillon-cylindre, soit $P_1$ le poids obtenu
- on calcule le pouvoir absorbant qui correspond à l'absorption surfacique grâce à la formule : Absorption $=4(P_1-P_0)/\pi D^2$ où D représente le diamètre du cylindre soit ici 0,0357 m.

D'où l'absorption exprimée en $g/m^2$ est définie ici par :

$$\text{Absorption} = (P_1-P_0)10^3.$$

Pour chaque test on réalise au moins 5 essais.

Le pouvoir absorbant obtenu est la moyenne de ces différents essais.

A partir de ces valeurs d'absorption on calcule la différence d'absorption entre les valeurs des formulations selon l'invention A(EXn) et les valeurs des formulations correspondantes sans ester d'acide gras de sorbitan éthoxylé A(ECn). D, exprimé en pourcentage, représente l'augmentation obtenue par rapport à l'échantillon sans ester d'acide gras de sorbitan éthoxylé.

$$D = \frac{A(EXn) - A(ECn)}{A(ECn)} \times 100$$

Les résultats obtenus ont été consignés dans les tableaux I, II et III.

**TABLEAU I**

| | EX1 | EC1 | EX2 | EC2 | EX3 | EC3 | EX4 | EC4 | EX5 | EC5 | EX6 | EC6 | EX7 | EC7 | EX8 | EC8 |
|---|---|---|---|---|---|---|---|---|---|---|---|---|---|---|---|---|
| A | 1760 | 1500 | 1670 | 1270 | 1170 | 1000 | 890 | 690 | 1190 | 950 | 1040 | 730 | 1900 | 1620 | 1250 | 1060 |
| D | 17,3 | | 31 | | 17 | | 29 | | 25,2 | | 42,5 | | 17,3 | | 18 | |
| G | 1000 | | 600 | | 400 | | 1000 | | 600 | | 400 | | 1000 | | 1000 | |
| CMC | 7H4XF | | 7H3XF | | A500 | | A500 | | 7H4XF | | 7H3XF | | 7H4XF | | 7H4XF | |

G : représente le grammage des rubans adhésifs utilisés pour la mesure de l'absorption exprimée en $g/m^2$.
A : représente l'absorption exprimée en $g/m^2$ à une heure.
D : représente la différence d'absorption à une heure entre les exemples A(EXn) et les exemples comparatifs A(ECn) par rapport à l'absorption de l'exemple comparatif A(ECn) correspondant exprimée en pourcentage.

**TABLEAU II**

| Utilisation de différents esters de sorbitan éthoxylé | | | | | |
|---|---|---|---|---|---|
| | EC1 | EX1 | EX9 | EX10 | EX11 |
| A | 1500 | 1760 | 1760 | 1710 | 1810 |
| D | | 17,3 | 17,3 | 14 | 20,6 |

D : représente la différence d'absorption à une heure entre les exemples A(EXn) et l'exemple comparatif A(ECI) par rapport à l'absorption de l'exemple comparatif A(ECI) exprimée en pourcentage.
A : représente l'absorption exprimée en $g/m^2$ à une heure
Tous les rubans adhésifs utilisés sont à 1000 $g/m^2$ sur un support en polyuréthanne

**TABLEAU III**

| Utilisation de différentes concentrations d'esters de sorbitan éthoxylé | | | | | | |
|---|---|---|---|---|---|---|
| | **EC1 EX14** | | **EC9 EX12** | | **EC9 EX13** | |
| A | 1500 | 1710 | 630 | 870 | 630 | 890 |
| D | 14 | | 38 | | 41,2 | |
| CMC | 35,7 | 35,7 | 30 | 30 | 30 | 30 |
| G | 1000 | 1000 | 600 | 600 | 600 | 600 |

CMC : représente le taux de BLANOSE®7H4XF, exprimé en pourcentage, présent dans la masse adhésive hydrocolloïde
G : représente le grammage des rubans adhésifs utilisés pour la mesure de l'absorption exprimée en $g/m^2$
A : représente l'absorption exprimée en $g/m^2$ à une heure.
D : représente la différence d'absorption à une heure entre l'exemple A(EXn) et l'exemple comparatif A(ECn) par rapport à l'absorption de l'exemple comparatif A(ECn) exprimée en pourcentage.
Tous les rubans adhésifs utilisés sont sur un support en polyuréthanne de 30 $\mu$m d'épaisseur.

L'analyse des résultats regroupés dans le tableau I illustre parfaitement l'action favorable du polysorbate 80 ou du polysorbate 65 dans le cas de l'exemple 8 sur l'augmentation de la capacité d'absorption dès la première heure des masses adhésives hydrocolloïdes selon l'invention.

On constate ainsi à la lecture de ce tableau que l'on a une différence importante du pourcentage d'absorption à une heure des masses adhésives hydrocolloïdes selon l'invention par rapport à des masses adhésives hydrocolloïdes sans polysorbate 80 puisque l'on a une augmentation D de l'ordre de 17 à 30 % et qui atteint même 42 % dans le cas de l'exemple 6.

On remarque aussi que l'on retrouve cette augmentation pour tous les grammages testés 1000, 600 et 400 $g/m^2$.

De même si on fait varier la nature de la carboxyméthylcellulose de sodium, granulométrie différente entre les exemples 1, 5, 7, 8 et les exemples 2, 6 ou potentiel absorbant différent de la carboxyméthylcellulose (exemple 3 et 4) on conserve toujours l'augmentation de l'absorption à une heure.

On constate aussi une augmentation de l'absorption pour des taux de carboxyméthylcellulose différents dans la masse adhésive hydrocolloïde. Il est ainsi de 35,7 % dans les exemples 1 à 6 et 8 et de 30 % dans l'exemple 7.

On retrouve aussi ce phénomène quelle que soit la nature de masse adhésive hydrocolloïde. On a ainsi des résultats comparables quelle que soit la carboxyméthylcellulose de sodium utilisée ou le grammage obtenu pour une masse adhésive hydrocolloïde composée à partir de copolymère poly(styrène-isoprène-styrène), résine tackifiante et

plastifiant avec un copolymère acrylate ayant une température de transition vitreuse inférieure à - 20 °C (exemples 1, 2 et 3) ou sans ce dernier (exemples 4, 5, 6 et 8). L'absence de ce composé ne modifie donc pas les résultats et l'augmentation de la capacité d'absorption dès la première heure due à l'ester de sorbitan éthoxylé.

De même on retrouve des résultats identiques avec une matrice adhésive à base de polyisobutylène de bas poids moléculaire et de copolymère séquencé poly(styrène-isoprène-styrène). Ainsi dans l'exemple 7, dont la matrice adhésive comprend 39,4 % de polyisobutylène, 15,3 % de copolymère séquencé poly(styrène-isoprène-styrène) et 8,1 % de polybutylène on a dès la première heure une augmentation du pouvoir d'absorption par rapport à une masse adhésive hydrocolloïde identique sans polysorbate 80 de 17,3 %.

De même on a un résultat analogue dans l'exemple 8 avec une matrice adhésive de nature différente à base de 3,95 % de polyisobutylène, 15,8 % de copolymère séquence poly(styrène-isoprène-styrène) et sans polyisobutylène mais avec une huile minérale.

L'analyse des résultats regroupés dans le tableau II illustre l'action favorable de divers esters de sorbitan éthoxylé sur l'augmentation de la capacité d'absorption dès la première heure des masses adhésives hydrocolloïdes selon l'invention.

On constate aussi à la lecture de ce tableau que cette augmentation est de l'ordre de 15 à 20 % quelle que soit la nature de l'ester utilisé (stéarate avec les polysorbates 60 et 65 ou oléate avec les polysorbates 80 et 81), le nombre d'unités w+x+y=z éthoxylées utilisées (5 avec le polysorbate 81 et 20 avec les polysorbates 65, 60 et 80) et le nombre d'esters présents (mono ester avec le polysorbate 60 et triesters avec le polysorbate 65).

Les résultats regroupés dans le tableau III illustrent l'action sur l'augmentation de l'absorption à 1 heure avec différentes concentrations d'esters de sorbitan éthoxylé. On peut ainsi remarquer que le polysorbate 65 (exemple 13) et le polysorbate 80 (exemple 12), utilisés avec un taux de carboxyméthylcellulose plus faible que dans le tableau II (30 % au lieu de 35,7 %) et présents dans ce cas à un taux de 1 % au lieu de 0,5 % dans le tableau II, augmente l'absorption de façon significative de l'ordre de 40 % par rapport à un produit (exemple comparatif 9) sans polysorbate.

L'exemple 14 qui ne contient que 0,1 % de polysorbate 80 présente une capacité d'absorption accrue de 14 %. Une fois de plus on retrouve des résultats positifs quelque soit le grammage (1000 g/$m^2$ dans l'exemple 14 et 600 g/$m^2$ dans les exemples 12 et 13).

On constate aussi au vu de ces résultats que les polysorbates permettent d'augmenter la capacité d'absorption dès la première heure à des proportions très faibles puisqu'ils sont efficaces à 0,5 % dans les exemples 1 à 11 et même à 0,1 % dans l'exemple 14. Ceci est un élément essentiel permettant de réaliser un pansement optimal sans détériorer les propriétés physiques (adhésion-cohésion-intégrité-élasticité).

On évite ainsi les problèmes de compatibilité qui peuvent se poser avec les autres composés de la masse adhésive hydrocolloïde. On obtient ainsi aisément un produit homogène, d'aspect correct et stable dans le temps.

Un autre avantage non négligeable de ces polysorbates est que ces composés sont déjà utilisés dans de nombreux secteurs industriels comme la pharmacie, la cosmétique ou l'alimentation humaine et animale dans lesquels on a démontré leur innocuité, leur stabilité, leur biodégradabilité et leur absence d'écotoxicité.

Leur utilisation dans la réalisation de pansements ne pose donc pas de problèmes de validation pharmaceutique car ils ne présentent aucun danger particulier.

En conclusion, toutes ces constatations et remarques démontrent de façon indéniable que l'addition d'esters de sorbitan éthoxylé tel le polysorbate 80 permet d'augmenter le pouvoir d'absorption des masses adhésives hydrocolloïdes dès les premières heures et les conséquences positives qui en résultent pour la réalisation de pansements pour le traitement des plaies, escarres, brûlures et lésions dermo-épidermiques superficielles, chroniques, profondes ou aiguës.

## Revendications

1. Masse adhésive hydrocolloïde notamment utilisable à des fins médicales **caractérisée en ce qu'**elle comprend :

   a) 0,2 à 5 parties en poids d'un ester d'acide gras de sorbitan éthoxylé,
   b) 20 à 50 parties en poids d'un hydrocolloïde
   c) 32 à 120 parties en poids d'une matrice adhésive constituée à partir d'un ou plusieurs polymères choisis parmi les copolymères séquencés poly(styrène-oléfine-styrène), les polyisobutylènes de bas poids moléculaire, les polyisobutylène de haut poids moléculaire, et un ou plusieurs composés choisis parmi les résines poisseuses dites tackifiantes, les plastifiants, les polybutènes, les antioxydants, les copolymères d'éthylène et d'acétate de vinyle, les caoutchoucs butyle et les copolymères blocs éthylène-propylène ; et,
   d) 0 à 15 parties en poids d'un polymère acrylate ayant une température de transition vitreuse inférieure à - 20 °C.

2. Masse hydrocolloïde selon la revendication 1, **caractérisée en ce qu'**elle comprend :

a) 0,2 à 5 parties en poids d'un ester d'acide gras de sorbitan éthoxylé,
b) 20 à 50 parties en poids d'un hydrocolloïde
c) 32 à 120 parties en poids d'une matrice adhésive constituée à partir d'un ou plusieurs polymères choisis parmi les copolymères séquencés poly(styrène-oléfine-styrène), les polyisobutylènes de bas poids moléculaire, les polyisobutylène de haut poids moléculaire, et un ou plusieurs composés choisis parmi les résines poisseuses dites tackifiantes, les plastifiants, les polybutènes, les antioxydants, les copolymères d'éthylène et d'acétate de vinyle, les caoutchoucs butyle et les copolymères blocs éthylène-propylène ; et,
d) 0,5 à 15 parties en poids d'un polymère acrylate ayant une température de transition vitreuse inférieure à -20 °C.

**3.** Masse adhésive hydrocolloïde selon la revendication 1 ou 2, **caractérisée en ce que** l'ester d'acide gras de sorbitan éthoxylé est choisi dans le groupe constitué par le polysorbate 20, le polysorbate 21, le polysorbate 40, le polysorbate 60, le polysorbate 61, le polysorbate 65, le polysorbate 80, le polysorbate 81, le polysorbate 85 et le polysorbate 120.

**4.** Masse adhésive hydrocolloïde selon la revendication 1 ou 2, **caractérisée en ce que** l'ester d'acide gras de sorbitan éthoxylé est un monoester de sorbitan éthoxylé, de préférence le polysorbate 80.

**5.** Masse adhésive hydrocolloïde selon l'une des revendications 2 à 4, **caractérisée en ce que** le polymère acrylate ayant une température de transition vitreuse inférieure à - 20 °C est un copolymère formé à partir d'au moins un monomère, choisi dans l'ensemble constitué par les esters alkylés de l'acide acrylique dans lesquels le groupe alkyle, linéaire ou ramifié, de l'ester comprend 1 à 18 atomes de carbone de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle, copolymérisé avec l'acide acrylique.

**6.** Masse adhésive hydrocolloïde selon la revendication 5, **caractérisée en ce que** le copolymère acrylate précité est un copolymère formé à partir d'au moins un monomère, choisi dans l'ensemble constitué par l'acrylate de n-butyle, l'acrylate de 2-éthylhexyle et l'acrylate d'isooctyle, copolymérisé avec l'acide acrylique et de préférence un copolymère de n-butylacrylate et d'acide acrylique ayant une température de transition vitreuse de -39 °C ou de n-butylacrylate, 2-éthylhexyle acrylate et d'acide acrylique ayant une température de transition vitreuse de -31 °C.

**7.** Masse adhésive hydrocolloïde selon la revendication 6, **caractérisée en ce que** le copolymère acrylate précité comprend de 1 à 20 %, de préférence 1 à 10 %, en poids d'acide acrylique exprimé par rapport au poids total de l'ensemble des monomères.

**8.** Masse adhésive hydrocolloïde selon l'une des revendications 2 à 4 **caractérisée en ce que** le polymère acrylate ayant une température de transition vitreuse inférieure à - 20 °C est un copolymère formé à partir d'au moins deux monomères, choisis dans l'ensemble constitué par les esters alkylés de l'acide acrylique dans lesquels le groupe alkyle, linéaire ou ramifié, de l'ester comprend 1 à 18 atomes de carbone, de préférence 4 à 10 atomes de carbone, en particulier 4 à 8 atomes de carbone, comme par exemple les acrylates de méthyle, d'éthyle, de n-propyle, de n-butyle, d'isobutyle, de n-hexyle, de 2-éthylhexyle, de n-octyle, d'isooctyle, de n-décyle et n-dodécyle.

**9.** Masse adhésive hydrocolloïde selon l'une des revendications 2 à 4, **caractérisée en ce que** le polymère acrylate ayant une température de transition vitreuse inférieure à -20 °C est un homopolymère dont le monomère constitutif est choisi dans l'ensemble constitué par les esters alkylés de l'acide acrylique dans lesquels le groupe alkyle de l'ester est soit un groupe alkyle linéaire qui comprend 2 à 12 atomes de carbone, soit un groupe isobutyle, 2-éthylhexyle ou isooctyle et de préférence un homopolymère d'acrylate de n-butyle ayant une température de transition vitreuse de -41 °C.

**10.** Masse adhésive hydrocolloïde selon la revendication 1,3 ou 4, **caractérisée en ce qu'**elle comprend :

a) 0,2 à 5 parties en poids d'un ester d'acide gras de sorbitan éthoxylé,
b) 20 à 50 parties en poids d'un hydrocolloïde
c) 32 à 120 parties en poids d'une matrice adhésive constituée à partir d'un ou plusieurs polymères choisis parmi les copolymères séquencés poly(styrène-oléfine-styrène), les polyisobutylènes de bas poids moléculaire, les polyisobutylène de haut poids moléculaire, et un ou plusieurs composés choisis parmi les résines poisseuses dites tackifiantes, les plastifiants, les polybutènes, les antioxydants, les copolymères d'éthylène et d'acétate de vinyle, les caoutchoucs butyle et les copolymères blocs éthylène-propylène.

**11.** Masse adhésive hydrocolloïde selon l'une des revendications 1 à 10 **caractérisée en ce que** la matrice adhésive de ladite masse adhésive hydrocolloïde est constituée de

- 10 à 35 parties en poids d'un copolymère séquencé poly(styrène-oléfine-styrène), en particulier poly(styrène-isoprène-styrène)
- 2 à 25 parties en poids d'un plastifiant, notamment une huile plastifiante
- 0,1 à 2 parties en poids d'au moins un antioxydant
- 20 à 50 parties en poids d'une résine tackifiante

**12.** Masse adhésive hydrocolloïde selon la revendication 11, **caractérisée en ce que** le plastifiant précité est une huile plastifiante minérale et de préférence une huile constituée de composés naphténiques, paraffiniques et aromatiques.

**13.** Masse adhésive hydrocolloïde selon l'une des revendications 1 à 10 **caractérisée en ce que** la matrice adhésive de ladite masse adhésive hydrocolloïde comprend un ou plusieurs polyisobutylènes de bas poids moléculaire compris entre 40 000 et 80 000 daltons.

**14.** Masse adhésive hydrocolloïde selon l'une des revendication 1 à 10, **caractérisée en ce que** la matrice adhésive de ladite masse adhésive hydrocolloïde comprend au moins un polyisobutylène de bas poids moléculaire et au moins un composé choisi parmi les polyisobutylènes de haut poids moléculaire, les polybutènes, les caoutchoucs butyle, les copolymères d'éthylène et d'acétate de vinyle, les copolymères blocs éthylène-propylène, et les copolymères séquencés poly(styrène-isoprène-styrène) ou poly(styrène-butadiène-styrène).

**15.** Masse adhésive hydrocolloïde selon la revendication 14, **caractérisée en ce que** la matrice adhésive de ladite masse adhésive hydrocolloïde comprend au moins un polyisobutylène de bas poids moléculaire et au moins un composé choisi parmi les polybutènes, les caoutchoucs butyle et les polyisobutylènes de haut poids moléculaire.

**16.** Masse adhésive hydrocolloïde selon la revendication 13 ou 14, **caractérisée en ce que** la matrice adhésive de ladite masse adhésive hydrocolloïde comprend au moins un polyisobutylène de bas poids moléculaire, un copolymère séquencé poly(styrène-oléfine-styrène) et un polybutène.

**17.** Masse adhésive hydrocolloïde selon la revendication 16, **caractérisée en ce qu'**elle comprend :

a) 5 à 20 parties en poids d'un copolymère séquencé poly(styrène-isoprène-styrène)
b) 25 à 50 parties en poids d'au moins un polyisobutylène de bas poids moléculaire
c) 2 à 20 parties en poids d'un polybutène
d) 20 à 50 parties en poids d'un hydrocolloïde
e) 0,2 à 5 parties en poids d'un monooléate de sorbitan éthoxylé
f) 0.5 à 15 parties en poids d'un polymère acrylate ayant une température de transition vitreuse inférieure à - 20 °C.
g) 0,1 à 2 parties en poids d'au moins un antioxydant.

**18.** Masse adhésive hydrocolloïde selon la revendication 1, **caractérisée en ce qu'**elle comprend, pour un total de 100 parties en poids :

a) 18 à 22, et de préférence 17,7 parties en poids d'un copolymère tribloc poly(styrène-isoprène-styrène),
b) 20 à 35, et de préférence 26,5 parties en poids d'une résine tackifiante,
c) 3 à 8, et de préférence 6,5 parties en poids d'un copolymère d'acrylate de n-butyle et d'acide acrylique qui présente une température de transition vitreuse de - 39 °C.
d) 10 à 20, et de préférence 12,4 parties en poids d'une huile plastifiante minérale,
e) 25 à 40, et de préférence 35,7 parties en poids de carboxyméthylcellulose de sodium,
f) 0,3 à 0,8, et de préférence 0,75 parties en poids d'un agent antioxydant phénolique,
0,3 à 0,8, et de préférence 0,35 parties en poids d'un agent antioxydant soufré, le dibutyldithiocarbamate de zinc, et,
g) 0,2 à 3 et de préférence 0,5 parties en poids de polysorbate 80.

**19.** Masse adhésive hydrocolloïde selon l'une des revendications 1 à 12, 14, 16 à 18, **caractérisée en ce que** le copolymère séquencé précité est un poly(styrène-isoprène-styrène) qui présente une teneur en styrène comprise entre 14 et 52 % en poids par rapport au poids dudit copolymère, et de préférence une teneur comprise entre 14 et 30 % en poids.

20. Masse adhésive hydrocolloïde selon l'une des revendications 1 à 19, **caractérisée en ce que** l'hydrocolloïde est un sel de métal alcalin de carboxyméthylcellulose, et de préférence la carboxyméthylcellulose de sodium.

21. Utilisation d'une masse adhésive hydrocolloïde suivant l'une quelconque des revendications 1 à 20 pour la préparation d'un pansement destiné au traitement de l'ampoule, des lésions dermo-épidermiques superficielles profondes, chroniques ou aiguës, exsudatives ou brûlures, formé d'un support sur lequel est enduit ladite masse adhésive hydrocolloïde et éventuellement d'une pellicule de protection pelable.

## Claims

1. Hydrocolloid adhesive mass useful especially for medical purposes, **characterized in that** it comprises:

   a) 0.2 to 5 parts by weight of an ethoxylated sorbitan fatty acid ester;
   b) 20 to 50 parts by weight of a hydrocolloid;
   c) 32 to 120 parts by weight of an adhesive matrix made up of one or more polymers selected from poly(styrene/olefin/styrene) block copolymers, low-molecular weight polyisobutylenes and high-molecular weight polyisobutylenes, and one or more compounds selected from sticky or tackifying resins, plasticizers, polybutenes, antioxidants, ethylene and vinyl acetate copolymers, butyl rubbers and ethylene-propylene block copolymers; and
   d) 0 to 15 parts by weight of an acrylate polymer with a glass transition temperature below -20°C.

2. Hydrocolloid mass according to claim 1, **characterized in that** it comprises:

   a) 0.2 to 5 parts by weight of an ethoxylated sorbitan fatty acid ester;
   b) 20 to 50 parts by weight of a hydrocolloid;
   c) 32 to 120 parts by weight of an adhesive matrix made up of one or more polymers selected from poly(styrene/olefin/styrene) block copolymers, low-molecular weight polyisobutylenes and high-molecular weight polyisobutylenes, and one or more compounds selected from sticky or tackifying resins, plasticizers, polybutenes, antioxidants, ethylene and vinyl acetate copolymers, butyl rubbers and ethylene-propylene block copolymers; and
   d) 0.5 to 15 parts by weight of an acrylate polymer with a glass transition temperature below -20°C.

3. Hydrocolloid adhesive mass according to claim 1 or 2, **characterized in that** the ethoxylated sorbitan fatty acid ester is selected from the group consisting of polysorbate 20, polysorbate 21, polysorbate 40, polysorbate 60, polysorbate 61, polysorbate 65, polysorbate 80, polysorbate 81, polysorbate 85 and polysorbate 120.

4. Hydrocolloid adhesive mass according to claim 1 or 2, **characterized in that** the ethoxylated sorbitan fatty acid ester is an ethoxylated sorbitan monoester, preferably polysorbate 80.

5. Hydrocolloid adhesive mass according to one of claims 2 to 4, **characterized in that** the acrylate polymer with a glass transition temperature below -20°C is a copolymer formed of at least one monomer selected from the group consisting of acrylic acid alkyl esters in which the linear or branched alkyl group of the ester contains 1 to 18 carbon atoms, preferably 4 to 10 carbon atoms and particularly 4 to 8 carbon atoms, for example methyl, ethyl, n-propyl, n-butyl, isobutyl, n-hexyl, 2-ethylhexyl, n-octyl, isooctyl, n-decyl and n-dodecyl acrylates, copolymerized with acrylic acid.

6. Hydrocolloid adhesive mass according to claim 5, **characterized in that** the above-mentioned acrylate copolymer is a copolymer formed of at least one monomer selected from the group consisting of n-butyl acrylate, 2-ethylhexyl acrylate and isooctyl acrylate, copolymerized with acrylic acid, and preferably an n-butyl acrylate/acrylic acid copolymer with a glass transition temperature of -39°C or an n-butyl acrylate/2-ethylhexyl acrylate/acrylic acid copolymer with a glass transition temperature of -31°C.

7. Hydrocolloid adhesive mass according to claim 6, **characterized in that** the above-mentioned acrylate copolymer comprises from 1 to 20% and preferably 1 to 10% by weight of acrylic acid, expressed relative to the total weight of all the monomers.

8. Hydrocolloid adhesive mass according to one of claims 2 to 4, **characterized in that** the acrylate polymer with a glass transition temperature below -20°C is a copolymer formed of at least two monomers selected from the group consisting of acrylic acid alkyl esters in which the linear or branched alkyl group of the ester contains 1 to 18 carbon

atoms, preferably 4 to 10 carbon atoms and particularly 4 to 8 carbon atoms, for example methyl, ethyl, n-propyl, n-butyl, isobutyl, n-hexyl, 2-ethylhexyl, n-octyl, isooctyl, n-decyl and n-dodecyl acrylates.

**9.** Hydrocolloid adhesive mass according to one of claims 2 to 4, **characterized in that** the acrylate polymer with a glass transition temperature below -20°C is a homopolymer whose constituent monomer is selected from the group consisting of acrylic acid alkyl esters in which the alkyl group of the ester is either a linear alkyl group containing 2 to 12 carbon atoms or an isobutyl, 2-ethylhexyl or isooctyl group, and preferably an n-butyl acrylate homopolymer with a glass transition temperature of -41°C.

**10.** Hydrocolloid adhesive mass according to claim 1, 3 or 4, **characterized in that** it comprises:

a) 0.2 to 5 parts by weight of an ethoxylated sorbitan fatty acid ester;
b) 20 to 50 parts by weight of a hydrocolloid; and
c) 32 to 120 parts by weight of an adhesive matrix made up of one or more polymers selected from poly(styrene/olefin/styrene) block copolymers, low-molecular weight polyisobutylenes and high-molecular weight polyisobutylenes, and one or more compounds selected from sticky or tackifying resins, plasticizers, polybutenes, antioxidants, ethylene and vinyl acetate copolymers, butyl rubbers and ethylene-propylene block copolymers.

**11.** Hydrocolloid adhesive mass according to one of claims 1 to 10, **characterized in that** the adhesive matrix of said hydrocolloid adhesive mass consists of:

- 10 to 35 parts by weight of a poly(styrene/olefin/styrene) block copolymer, particularly poly(styrene/isoprene/styrene);
- 2 to 25 parts by weight of a plasticizer, especially a plasticizing oil;
- 0.1 to 2 parts by weight of at least one antioxidant; and
- 20 to 50 parts by weight of a tackifying resin.

**12.** Hydrocolloid adhesive mass according to claim 11, **characterized in that** the above-mentioned plasticizer is a mineral plasticizing oil and preferably an oil consisting of naphthenic, paraffinic and aromatic compounds.

**13.** Hydrocolloid adhesive mass according to one of claims 1 to 10, **characterized in that** the adhesive matrix of said hydrocolloid adhesive mass comprises one or more polyisobutylenes with a low molecular weight of between 40,000 and 80,000 daltons.

**14.** Hydrocolloid adhesive mass according to one of claims 1 to 10, **characterized in that** the adhesive matrix of said hydrocolloid adhesive mass comprises at least one low-molecular weight polyisobutylene and at least one compound selected from high-molecular weight polyisobutylenes, polybutenes, butyl rubbers, ethylene and vinyl acetate copolymers, ethylene-propylene block copolymers and poly(styrene/isoprene/styrene) or poly(styrene/butadiene/styrene) block copolymers.

**15.** Hydrocolloid adhesive mass according to claim 14, **characterized in that** the adhesive matrix of said hydrocolloid adhesive mass comprises at least one low-molecular weight polyisobutylene and at least one compound selected from polybutenes, butyl rubbers and high-molecular weight polyisobutylenes.

**16.** Hydrocolloid adhesive mass according to claim 13 or 14, **characterized in that** the adhesive matrix of said hydrocolloid adhesive mass comprises at least one low-molecular weight polyisobutylene, one poly(styrene/olefin/styrene) block copolymer and one polybutene.

**17.** Hydrocolloid adhesive mass according to claim 16, **characterized in that** it comprises:

a) 5 to 20 parts by weight of a poly(styrene/isoprene/styrene) block copolymer;
b) 25 to 50 parts by weight of at least one low-molecular weight polyisobutylene;
c) 2 to 20 parts by weight of a polybutene;
d) 20 to 50 parts by weight of a hydrocolloid;
e) 0.2 to 5 parts by weight of an ethoxylated sorbitan monooleate;
f) 0.5 to 15 parts by weight of an acrylate polymer with a glass transition temperature below -20°C; and
g) 0.1 to 2 parts by weight of at least one antioxidant.

**18.** Hydrocolloid adhesive mass according to claim 1, **characterized in that** it comprises, for a total of 100 parts by weight:

a) 18 to 22 and preferably 17.7 parts by weight of a poly(styrene/isoprene/styrene) three-block copolymer;
b) 20 to 35 and preferably 26.5 parts by weight of a tackifying resin;
c) 3 to 8 and preferably 6.5 parts by weight of an n-butyl acrylate and acrylic acid copolymer with a glass transition temperature of -39°C;
d) 10 to 20 and preferably 12.4 parts by weight of a mineral plasticizing oil;
e) 25 to 40 and preferably 35.7 parts by weight of sodium carboxymethyl cellulose;
f) 0.3 to 0.8 and preferably 0.75 part by weight of a phenolic antioxidant and 0.3 to 0.8 and preferably 0.35 part by weight of the sulfur-containing antioxidant zinc dibutyldithiocarbamate; and
g) 0.2 to 3 and preferably 0.5 part by weight of polysorbate 80.

**19.** Hydrocolloid adhesive mass according to one of claims 1 to 12, 14 and 16 to 18, **characterized in that** the above-mentioned block copolymer is a poly(styrene/isoprene/styrene) with a styrene content of between 14 and 52% by weight, based on the weight of said copolymer, and preferably a content of between 14 and 30% by weight.

**20.** Hydrocolloid adhesive mass according to one of claims 1 to 19, **characterized in that** the hydrocolloid is an alkali metal salt of carboxymethyl cellulose and preferably sodium carboxymethyl cellulose.

**21.** Use of a hydrocolloid adhesive mass according to any one of claims 1 to 20 for the preparation of a dressing for the treatment of blisters, superficial, deep, chronic, acute or exudative dermo-epidermal lesions or bums, formed of a support onto which said hydrocolloid adhesive mass is coated, and optionally of a peel-off protective film.

## Patentansprüche

**1.** Hydrokolloidale Klebemasse, die insbesondere zu medizinischen Zwecken verwendbar ist, **dadurch gekennzeichnet, daß** sie umfaßt:

a) 0,2 bis 5 Gewichtsteile eines Fettsäureesters von ethoxyliertem Sorbitan,
b) 20 bis 50 Gewichtsteile eines Hydrokolloids,
c) 32 bis 120 Gewichtsteile einer adhäsiven Matrix, gebildet ausgehend von einem oder mehreren Polymeren, gewählt unter den sequentiellen Copolymeren Poly-(Styrol-Olefin-Styrol), den Polyisobutylenen niedrigen Molekulargewichts, den Polyisobutylenen hohen Molekulargewichts, und einer oder mehrerer Verbindungen, gewählt aus den Klebrigmacher genannten klebrigen Harzen, den Weichmachern, den Polybutenen, den Antioxidationsmitteln, den Copolymeren von Ethylen und Vinylacetat, den Butylkautschuken und den Ethylen-Propylen-Blockcopolymeren, und
d) 0 bis 15 Gewichtsteile eines Acrylatpolymers mit einer Glasübergangstemperatur unter -20°C.

**2.** Hydrokolloidale Masse nach Anspruch 1, **dadurch gekennzeichnet, daß** sie umfaßt:

a) 0,2 bis 5 Gewichtsteile eines Fettsäureesters von ethoxyliertem Sorbitan,
b) 20 bis 50 Gewichtsteile eines Hydrokolloids,
c) 32 bis 120 Gewichtsteile einer adhäsiven Matrix, gebildet ausgehend von einem oder mehreren Polymeren, gewählt unter den sequentiellen Copolymeren Poly-(Styrol-Olefin-Styrol), den Polyisobutylenen niedrigen Molekulargewichts, den Polyisobutylenen hohen Molekulargewichts und einer oder mehrerer Verbindungen, gewählt aus den Klebrigmacher genannten klebrigen Harzen, den Weichmachern, den Polybutenen, den Antioxidationsmitteln, den Copolymeren von Ethylen und Vinylacetat, den Butylkautschuken und den Ethylen-Propylen-Blockcopolymeren, und
d) 0 bis 15 Gewichtsteile eines Acrylatpolymers mit einer Glasübergangstemperatur unter -20°C.

**3.** Hydrokolloidale Klebemasse nach Anspruch 1 oder 2, **dadurch gekennzeichnet, daß** der Fettsäureester von ethoxyliertem Siloxan gewählt ist aus der Gruppe, die besteht aus Polysorbat 20, Polysorbat 21, Polysorbat 40, Polysorbat 60, Polysorbat 61, Polysorbat 65, Polysorbat 80, Polysorbat 81, Polysorbat 85 und Polysorbat 120.

**4.** Hydrokolloidale Klebemasse nach Anspruch 1 oder 2, **dadurch gekennzeichnet, daß** der Fettsäureester von ethoxyliertem Sorbitan ein ethoxylierter Sorbitanmonoester, vorzugsweise Polysorbat 80 ist.

**5.** Hydrokolloidale Klebemasse nach einem der Ansprüche 2 bis 4, **dadurch gekennzeichnet, daß** das Acrylatpolymer mit einer Glasübergangstemperatur unter -20°C ein Copolymer ist, gebildet ausgehend von wenigstens einem Monomer, gewählt aus der Gesamtheit, die besteht aus den Alkylestern von Acrylsäure, bei denen die Alkylgruppe linear oder verzweigt ist, wobei der Ester 1 bis 18 Kohlenstoffatome, vorzugsweise 4 bis 10 Kohlenstoffatome umfaßt, insbesondere 4 bis 8 Kohlenstoffatome, wie zum Beispiel die Methyl-, Ethyl-, n-Propyl-, n-Butyl-, Isobutyl-, n-Hexyl-, 2-Ethylhexyl-, n-Octyl-, Isooctyl-, n-Decyl- und n-Dodecylacrylate, das copolymerisiert ist mit Acrylsäure.

**6.** Hydrokolloidale Klebemasse nach Anspruch 5, **dadurch gekennzeichnet, daß** das genannte Acrylatcopolymer ein Copolymer ist, gebildet ausgehend von wenigstens einem Monomer, gewählt aus der Gesamtheit, die besteht aus n-Butylacrylat, 2-Ethylhexylacrylat und Isooctylacrylat, copolymerisiert mit Acrylsäure und vorzugsweise ein Copolymer von n-Butylacrylat und Acrylsäure mit einer Glasübergangstemperatur von -39°C oder n-Butylacrylat, 2-Ethylhexylacrylat und Acrylsäure mit einer Glasübergangstemperatur von -31°C.

**7.** Hydrokolloidale Klebemasse nach Anspruch 6, **dadurch gekennzeichnet, daß** das genannte Acrylatcopolymer 1 bis 20, vorzugsweise 1 bis 10 Gewichtsprozent Acrylsäure, ausgedrückt im Verhältnis zum Gesamtgewicht der Gesamtheit der Monomere umfaßt.

**8.** Hydrokolloidale Klebemasse nach einem der Ansprüche 2 bis 4, **dadurch gekennzeichnet, daß** das Acrylatpolymer mit einer Glasübergangstemperatur bei -20°C ein Copolymer ist, das gebildet ist aus wenigstens zwei Monomeren, gewählt aus der Gesamtheit, die besteht aus den Alkylestern von Acrylsäure, bei denen die Alkylgruppe linear oder verzweigt ist und der Ester 1 bis 18 Kohlenstoffatome, vorzugsweise 4 bis 10 Kohlenstoffatome, insbesondere 4 bis 8 Kohlenstoffatome umfaßt, wie zum Beispiel die Methyl-, Ethyl-, n-Propyl-, n-Butyl-, Isobutyl-, n-Hexyl-, 2-Ethylhexyl-, n-Octyl-, Isooctyl-, n-Decyl- und n-Dodecylacrylate.

**9.** Hydrokolloidale Klebemasse nach einem der Ansprüche 2 bis 4, **dadurch gekennzeichnet, daß** das Acrylatpolymer mit einer Glasübergangstemperatur unter -20°C ein Homopolymer ist, dessen Bestandteilmonomer gewählt ist aus der Gesamtheit, die besteht aus den Alkylestern von Acrylsäure, in denen die Alkylgruppe des Esters entweder eine lineare Alkylgruppe, die 2 bis 12 Kohlenstoffatome umfaßt, oder eine Isobutyl-, 2-Ethylhexyl- oder Isooctylgruppe und vorzugsweise ein Homopolymer von n-Butylacrylat mit einer Glasübergangstemperatur von -41°C ist.

**10.** Hydrokolloidale Klebemasse nach Anspruch 1, 3 oder 4, **dadurch gekennzeichnet, daß** sie umfaßt:

a) 0,2 bis 5 Gewichtsteile eines Fettsäureesters von ethoxyliertem Sorbitan,
b) 20 bis 50 Gewichtsteile eines Hydrokolloids,
c) 32 bis 120 Gewichtsteile einer adhäsiven Matrix, gebildet ausgehend von einem oder mehreren Polymeren, gewählt unter den sequentiellen Copolymeren Poly-(Styrol-Olefin-Styrol), den Polyisobutylenen niedrigen Molekulargewichts, den Polyisobutylenen hohen Molekulargewichts, und einer oder mehrerer Verbindungen, gewählt aus den Klebrigmacher genannten klebrigen Harzen, den Weichmachern, den Polybutenen, den Antioxidationsmitteln, den Copolymeren von Ethylen und Vinylacetat, den Butylkautschuken und den Ethylen-Propylen-Blockcopolymeren.

**11.** Hydrokolloidale Klebemasse nach einem der Ansprüche 1 bis 10, **dadurch gekennzeichnet, daß** die adhäsive Matrix der hydrokolloidalen Klebemasse besteht aus:

- 10 bis 35 Gewichtsteilen eines sequentiellen Copolymers Poly-(Styrol-Olefin-Styrol), insbesondere Poly-(Styrol-Isopren-Styrol),
- 2 bis 25 Gewichtsteile eines Weichmachers, insbesondere eines Weichmacheröls,
- 0,1 bis 2 Gewichtsteile wenigstens eines Antioxidationsmittels,
- 20 bis 50 Gewichtsteile eines klebrigmachenden Harzes.

**12.** Hydrokolloidale Klebemasse nach Anspruch 11, **dadurch gekennzeichnet, daß** der genannte Weichmacher ein Weichmachermineralöl und vorzugsweise ein Öl ist, das aus naphtenischen, paraffinischen und aromatischen Verbindungen besteht.

**13.** Hydrokolloidale Klebemasse nach einem der Ansprüche 1 bis 10, **dadurch gekennzeichnet, daß** die adhäsive Matrix der hydrokolloidalen Klebemasse eine oder mehrere Polyisobutylene niedrigen Molekulargewichts zwischen 40.000 und 80.000 Dalton umfaßt.

**14.** Hydrokolloidale Klebemasse nach einem der Ansprüche 1 bis 10, **dadurch gekennzeichnet, daß** die adhäsive Matrix der hydrokolloidalen Klebemasse wenigstens ein Polyisobutylen niedrigen Molekulargewichts und wenigstens eine Verbindung umfaßt, gewählt unter den Polyisobutylenen hohen Molekulargewichts, den Polybutenen, den Butylkautschuken, den Copolymeren von Ethylen und Vinylacetat, den Ethylen-Propylen-Blockcopolymeren und den sequentiellen Copolymeren Poly-(Styrol-Isopren-Styrol) oder Poly-(Styrol-Butadien-Styrol).

**15.** Hydrokolloidale Klebemasse nach Anspruch 14, **dadurch gekennzeichnet, daß** die adhäsive Matrix der hydrokolloidalen Klebemasse wenigstens ein Polyisobutylen niedrigen Molekulargewichts und wenigstens eine Verbindung umfaßt, die gewählt ist unter den Polybutenen, den Butylkautschuken und den Polyisobutylenen hohen Molekulargewichts.

**16.** Hydrokolloidale Klebemasse nach Anspruch 13 oder 14, **dadurch gekennzeichnet, daß** die adhäsive Matrix der hydrokolloidalen Klebemasse wenigstens ein Polyisobutylen niedrigen Molekulargewichts, ein sequentielles Copolymer Poly-(Styrol-Olefin-Styrol) oder Polybutylen umfaßt.

**17.** Hydrokolloidale Klebemasse nach Anspruch 16, **dadurch gekennzeichnet, daß** sie umfaßt:

a) 5 bis 20 Gewichtsteile wenigstens eines sequentiellen Copolymers Poly-(Styrol-Isopren-Styrol),
b) 25 bis 50 Gewichtsteile wenigstens eines Polyisobutylens niedrigen Molekulargewichts,
c) 2 bis 20 Gewichtsteile eines Polybutens,
d) 20 bis 50 Gewichtsteile eines Hydrokolloids,
e) 0,2 bis 5 Gewichtsteile eines Monooleats von ethoxyliertem Sorbitan,
f) 0,5 bis 15 Gewichtsteile eines Acrylatpolymers mit einer Glasübergangstemperatur unter -20°C,
g) 0,1 bis 2 Gewichtsteile wenigstens eines Antioxidationsmittels.

**18.** Hydrokolloidale Klebemasse nach Anspruch 1, **dadurch gekennzeichnet, daß** sie für eine Gesamtheit von 100 Gewichtsteilen umfaßt:

a) 18 bis 22, vorzugsweise 17,7 Gewichtsteile eines Triblockcopolymers Poly-(Styrol- Isopren-Styrol),
b) 20 bis 35 und vorzugsweise 26,5 Gewichtsteile eines klebrigmachenden Harzes,
c) 3 bis 8, vorzugsweise 6,5 Gewichtsteile eines Copolymers von n-Butylacrylat und Acrylsäure, das eine Glasübergangstemperatur von -39°C aufweist,
d) 10 bis 20 und vorzugsweise 12,4 Gewichtsteile eines Mineralölweichmachers,
e) 25 bis 40, vorzugsweise 35,7 Gewichtsteile Natriumcarboxymethylcellulose,
f) 0,3 bis 0,8 und vorzugsweise 0,75 Gewichtsteile eines Phenolantioxidationsmittels,
0,3 bis 0,8 und vorzugsweise 0,35 Gewichtsteile eines Schwefelantioxidationsmittels, Zinkdibutyldithiocarbamat und
g) 0,2 bis 3 und vorzugsweise 0,5 Gewichtsteile Polysorbat 80.

**19.** Hydrokolloidale Klebemasse nach einem der Ansprüche 1 bis 12, 14, 16 bis 18, **dadurch gekennzeichnet, daß** das genannte sequentielle Copolymer ein Poly-(Styrol-Isopren-Styrol) ist, das einen Styrolgehalt zwischen 14 und 52 Gewichtsprozent im Verhältnis zum Gewicht des Copolymeres und vorzugsweise einen Gehalt zwischen 14 und 30 Gewichtsprozent umfaßt.

**20.** Hydrokolloidale Klebemasse nach einem der Ansprüche 1 bis 19, **dadurch gekennzeichnet, daß** das Hydrokolloid ein Alkalimetallsalz von Carboxylmethylcellulose ist und vorzugsweise Natriumcarboxymethylcellulose.

**21.** Verwendung einer hydrokolloidalen Klebemasse nach einem der Ansprüche 1 bis 20 zur Herstellung eines Pflasters, das vorgesehen ist zur Behandlung einer Blase, von tiefgreifenden Dermis-Epidermis-Oberflächenverletzungen, chronisch oder akut, Exsudativen oder Verbrennungen und das gebildet aus einem Träger, auf den die hydrokolloidale Klebemasse getränkt wird, und gegebenenfalls einem abschälbaren Schutzfilm.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*


### Documents brevets cités dans la description

- US 3972328 A
- FR 2495473 A
- EP 130061 A
- EP 302536 A

### Littérature non-brevet citée dans la description

- Advances in Pressure Sensitive Adhesive Technology-2. *Wound Dressings,* Avril 1995, 158-171